# EUROPEAN PATENT APPLICATION

(11) **EP 3 714 776 A1**
(43) Date of publication of application: **30.09.2020**
(21) Application number: 18880783.8
(22) Date of filing: 01.11.2018
(51) Int. Cl.: A61B 5/02, A61B 5/0245

(54) **ELECTRONIC DEVICE**

(30) Priority: 22.11.2017 JP 2017225141
(71) Applicant: Kyocera Corporation, Kyoto-shi, Kyoto 612-8501 (JP)
(72) Inventor: AJIMA Hiromi, Kyoto-shi Kyoto 612-8501 (JP)
(74) Representative: Viering, Jentschura & Partner mbB Patent- und Rechtsanwälte
(86) International application number: PCT/JP2018/040699
(87) International publication number: WO 2019/102815

(57) **Abstract**

An electronic device includes a base and a meter that can be displaced along a plane intersecting a surface of the base. The meter includes an arm that can be displaced in a direction approximately parallel to a displacement direction of the meter, and a sensor capable of detecting displacement of the arm in accordance with the pulse wave.

## Description

### CROSS REFERENCE TO RELATED APPLICATION

This application claims priority to and the benefit of Japanese Patent Application No. 2017-225141 filed on November 22, 2017, the entire contents of which are incorporated herein by reference.

### TECHNICAL FIELD

The present disclosure relates to an electronic device.

### BACKGROUND

Conventionally, electronic devices that acquire biological information of a subject in a state being worn on the wrist of the subject are known (e.g., see PTL 1 and PTL 2).

### CITATION LIST

### Patent Literature

PTL 1: WO 2016/174839 A1
PTL 2: WO 2016/194308 A1

### SUMMARY

One aspect of an electronic device includes a base and a meter that can be displaced along a plane intersecting a surface of the base. The meter includes an arm that can be displaced in a direction approximately parallel to a displacement direction of the meter in accordance with a pulse wave of a subject, and a sensor capable of detecting a displacement of the arm in accordance with the pulse wave.

### BRIEF DESCRIPTION OF THE DRAWINGS

FIG. 1 is a schematic perspective view illustrating an exterior of an electronic device according to an embodiment;
FIG. 2 is a schematic diagram illustrating a state in which the electronic device illustrated in FIG. 1 is being worn;
FIG. 3 is a schematic diagram illustrating an exterior portion and a sensor in an elevation view of the electronic device illustrated in FIG. 1;
FIG. 4 is a schematic diagram illustrating a positional relationship between the wrist of a subject and a first arm of the sensor in an elevation view;
FIG. 5A is a schematic diagram illustrating a positional relationship between the wrist of the subject, the first arm of the sensor, and the exterior portion of a meter;
FIG. 5B is a schematic diagram illustrating a positional relationship between the wrist of the subject, the first arm of the sensor, and the exterior portion of the meter;
FIG. 6 is a functional block diagram illustrating a schematic configuration of the electronic device illustrated in FIG. 1;
FIG. 7 is a graph illustrating an example of a pulse wave acquire by the sensor;
FIG. 8 is a graph illustrating a temporal variation of a calculated AI;
FIG. 9 is a graph illustrating the calculated AI and a blood glucose level measurement result;
FIG. 10 is a graph illustrating a relationship between the calculated AI and the blood glucose level;
FIG. 11 is a graph illustrating the calculated AI and a triglyceride level measurement result;
FIG. 12 is a flowchart illustrating a procedure for estimating blood fluidity, a glucose metabolism condition, and a lipid metabolism condition;
FIG. 13 is a diagram illustrating a schematic configuration of a system according to an embodiment;
FIG. 14 is a schematic diagram illustrating an example variation of the positional relationship between the wrist of the subject, the first arm of the sensor, and the exterior portion of the meter in the elevation view; and
FIG. 15 is a schematic diagram illustrating a positional relationship between the wrist of the subject and the first arm of the sensor in the elevation view.

### DETAILED DESCRIPTION

It can be hard for an electronic device to accurately acquire biological information, depending on its state being worn. An electronic device configured to facilitate more accurate acquisition of the biological information improves usability for a subject. The present disclosure relates to providing an electronic device capable of improving usability. According to one embodiment, an electronic device capable of improving usability can be provided. Hereinafter, the embodiment will be described in detail with reference to the accompanying drawings.

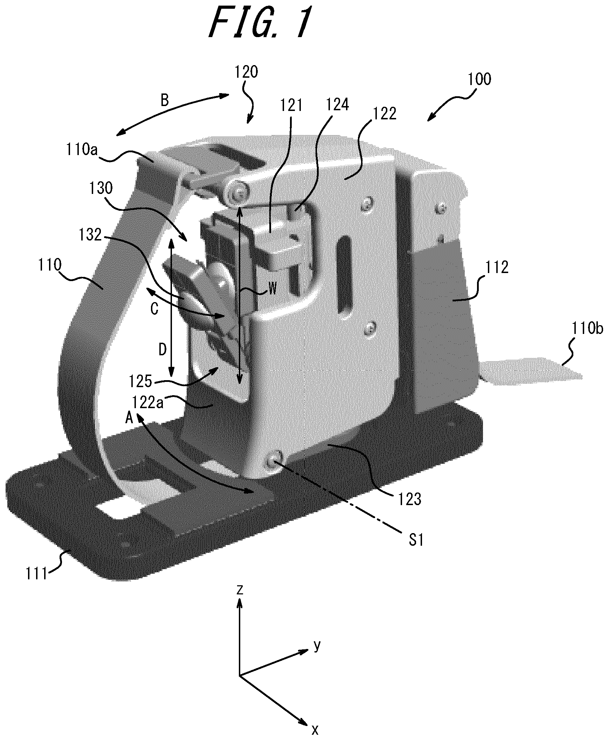

FIG. 1 is a schematic perspective view illustrating an exterior of an electronic device 100 according to the embodiment. The electronic device 100 includes a wearing portion 110, a base 111, and a fixing unit 112 attached to the base 111, and a meter 120.

According to the present embodiment, the base 111 has an approximately rectangular flat plate-like shape. Hereinafter, an x-axis direction corresponds to a transverse direction of the base 111 that has the approximately rectangular plate-like shape, a y-axis direction corresponds to a longitudinal direction of the base 111, and a z-axis direction corresponds to a direction orthogonal to the base 111. The electronic device 100 is partially movable as described herein, and directions mentioned herein relative to the electronic device 100 will refer to the x, y, and z directions in the state illustrated in FIG. 1, unless otherwise specified. Also, a positive z-axis direction as used herein corresponds to the upward direction, a negative z-axis direction as used herein corresponds to the downward direction, and a positive x-axis direction as used herein corresponds to a front side of the electronic device 100.

The electronic device 100 measures biological information of a subject when the electronic device 100 is worn by a subject using the wearing portion 110. The biological information to be measured by the electronic device 100 is a pulse wave of the subject that can be measured by the meter 120. In the present embodiment, the electronic device 100 will be described as being configured to acquire a pulse wave when the subject is wearing the electronic device 100 on the wrist, by way of example.

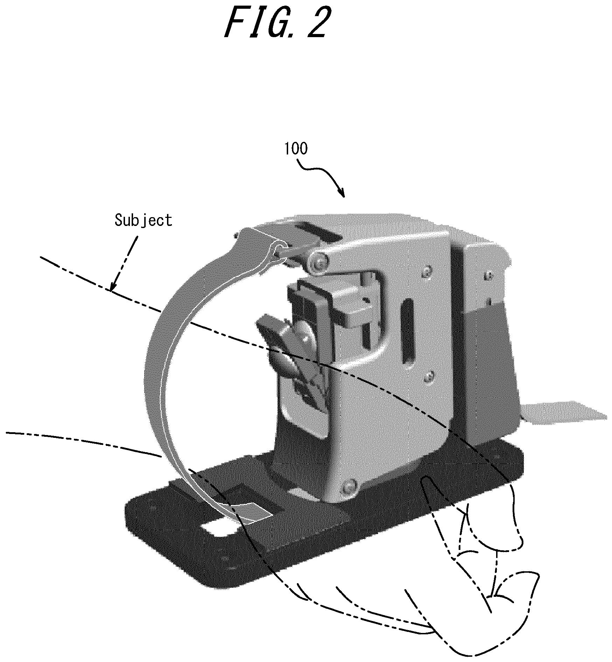

FIG. 2 is a schematic diagram illustrating a state in which the subject is wearing the electronic device 100 illustrated in FIG. 1. The subject can wear the electronic device 100 in a manner as illustrated in FIG. 2 by inserting the wrist through a space formed by the wearing portion 110, the base 111, and the meter 120 and stabilizing the wrist using the wearing portion 110. In the example illustrated in FIG. 1 and FIG. 2, the subject wears the electronic device 100 by inserting the wrist into the space formed by the wearing portion 110, the base 111, and the meter 120 in the positive x-axis direction along the x-axis direction. For example, the subject wears the electronic device 100 such that a pulse contact portion 132 of the meter 120, which will be described later, contacts a position where the ulnar artery or the radial artery exists. The electronic device 100 measures a pulse wave of the blood flowing through the ulnar artery or the radial artery in the wrist of the subject.

The meter 120 includes a main body 121, an exterior portion 122, and a sensor 130. The sensor 130 is attached to the main body 121. The meter 120 is attached to the base 111 via a connecting portion 123.

The connecting portion 123 may be attached to the base 111 in a manner to be able to rotate along the surface of the base 111 with respect to the base 111. In the example illustrated in FIG. 1, that is, the connecting portion 123 may be attached to the base 111 in a manner to be able to rotate on an xy plane with respect to the base 111 as indicated by an arrow A. In this case, the meter 120 attached to the base 111 via the connecting portion 123 can rotate in its entirety on the xy plane with respect to the base 111.

The exterior portion 122 is connected to the connecting portion 123 on an axis S1 that passes through the connecting portion 123. The axis S1 is an axis extending in the x-axis direction. Such connection of the exterior portion 122 to the connecting portion 123 in this manner enables the exterior portion 122 to be displaced along a plane intersecting the xy plane in which the base 111 extends, with respect to the connecting portion 123. That is, the exterior portion 122 can be inclined at a prescribed angle about the axis S1 on the xy plane in which the base 111 extends. For example, the exterior portion 122 can be displaced in a state sitting on a plane such as a yz plane having a predetermined inclination with respect to the xy plane. According to the present embodiment, the exterior portion 122 may be connected to the connecting portion 123 in a manner to be able to rotate about the axis S1 on the yz plane orthogonal to the xy plane, as indicated by an arrow B in FIG. 1.

The exterior portion 122 includes a contact surface 122a that comes into contact with the wrist of the subject when the electronic device 100 is worn. The exterior portion 122 may include an opening 125 on the same side as the contact surface 122a. The exterior portion 122 may be configured to cover the main body 121.

The exterior portion 122 may include a shaft 124 that extends in the z-axis direction within an inner space thereof. The main body 121 has an opening into which the shaft 124 is inserted and, in a state in which the shaft 124 is inserted into the opening, the main body 121 is arranged in the inner space of the exterior portion 122. That is, the main body 121 is attached to the exterior portion 122 in a manner to be able to rotate about the shaft 124 on the xy plane with respect to the exterior portion 122, as indicated by an arrow C illustrated in FIG. 1. In other words, the main body 121 is attached to the exterior portion 122 in a manner to be able to rotate along the xy plane serving as a surface of the base 111 with respect to the exterior portion 122. Further, the main body 121 is attached to the exterior portion 122 in a manner to be able be displaced in the up-down direction with respect to the exterior portion 122 along the shaft 124, i.e., along the z-axis direction, as indicated by an arrow D illustrated in FIG. 1.

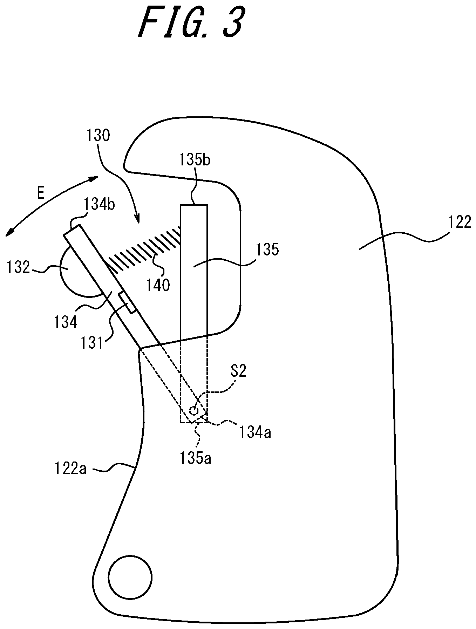

The sensor 130 is attached to the main body 121. Here, the sensor 130 will be described in detail with reference to FIG. 3. FIG. 3 is a schematic diagram illustrating the exterior portion 122 and the sensor 130 in an elevation view of the electronic device 100. Portions of the sensor 130 overlapping with the exterior portion 122 in the elevation view are represented by broken lines in FIG. 3.

The sensor 130 includes a first arm 134 and a second arm 135. The second arm 135 is fixed to the main body 121. A first end 135a of the second arm 135 is connected to a first end 134a of the first arm 134. The first arm 134 is connected to the second arm 135 in such a manner that a portion of the first arm 134 in the vicinity of a second end 134b can rotate about the first end 134a on the yz plane, as indicated by an arrow E in FIG. 3.

The portion of the first arm 134 in the vicinity of the second end 134b is connected to a portion of the second arm 135 in the vicinity of a second end 135b via an elastic member 140. In a state in which the elastic member 140 is not pressed, the first arm 134 is supported by the second arm 135 in such a manner that the second end 134b of the sensor 130 protrudes toward the contact surface 122a from the opening 125 of the exterior portion 122. The elastic member 140 is, for example, a spring. However, the elastic member 140 is not limited to a spring and may be any other elastic member such as, for example, a resin or a sponge.

A pulse contact portion 132 is attached to the second end 134b of the first arm 134. The pulse contact portion 132 is a member that comes into contact with a measured part of the subject for the measurement of a pulse wave of the blood when the electronic device 100 is worn. According to the present embodiment, the pulse contact portion 132 contacts, for example, a position where the ulnar artery or the radial artery exists. The pulse contact portion 132 may be formed from a material that is not likely to absorb a change occurred on the body surface due to the pulse of the subject. The pulse contact portion 132 may be formed from a material that does not cause pain to the subject when being in contact with the subject. For example, the pulse contact portion 132 may be formed by a cloth bag filled with beads. For example, the pulse contact portion 132 may be detachably attached to the first arm 134. For example, the subject may select and wear one pulse contact portion 132 from a plurality of pulse contact portions 132, in accordance with the size and/or shape of the wrist of the subject. In this way, the subject can use the pulse contact portion 132 that matches the size and/or shape of the wrist of the subject.

The sensor 130 includes an angular velocity sensor 131 for detecting displacement of the first arm 134. The angular velocity sensor 131 needs to simply detect an angular displacement of the first arm 134. The sensor 130 is not limited to include the angular velocity sensor 131 and may include, for example, an acceleration sensor, an angle sensor, other motion sensors, or any combination thereof.

According to the present embodiment, when the electronic device 100 is worn, the pulse contact portion 132 contacts the skin above the radial artery that runs on the thumb side of the right hand of the subject, as illustrated in FIG. 2. Because of the elastic force of the elastic member 140 provided between the second arm 135 and the first arm 134, the pulse contact portion 132 arranged in the vicinity of the second end 134b of the first arm 134 comes into contact with the skin above the radial artery of the subject. The first arm 134 is displaced in accordance with the movement of the radial artery of the subject, i.e., the pulsation. The angular velocity sensor 131 acquires a pulse wave by detecting the displacement of the first arm 134. The pulse wave is in the form of a wave representing temporal volume changes in the blood vessel caused by the inflow of the blood captured from the body surface.

In a state in which the elastic member 140 is not pressed, the second end 134b of the first arm 134 protrudes from the opening 125, as illustrated in FIG. 3. When the subject wears the electronic device 100, the pulse contact portion 132 attached to the first arm 134 contacts the skin above the radial artery of the subject. The elastic member 140 expands and contracts in accordance with the pulsation, and thus the pulse contact portion 132 is displaced. The elastic member 140 has an appropriate elasticity to be able to contract and expand in accordance with the pulsation without interfering with the pulsation. An opening width W of the opening 125 is sufficiently larger than a diameter of the blood vessel, i.e., the diameter of the radial artery in the present embodiment. By virtue of the opening 125 provided to the exterior portion 122, the contact surface 122a of the exterior portion 122 does not compress the radial artery when the electronic device 100 is worn. Thus, the electronic device 100 can acquire a pulse wave that includes less noises, and improve measurement accuracy.

The fixing unit 112 is fixed to the base 111. The fixing unit 112 may have a locking mechanism for securing the wearing portion 110. The wearing portion 110 may include various functional units used for the measurement of the pulse wave by the electronic device 100. For example, the wearing portion 110 may include a controller, which will be described later, a power source, a memory, a communication interface, a notification interface, a circuit for operating them, a cable connecting them, and the like.

The wearing portion 110 is a mechanism used by the subject to fix the electronic device 100 on the wrist. In the example illustrated in FIG. 1, the wearing portion 110 is an elongated strip-like band. In the example shown in FIG. 1, the wearing portion 110 is arranged such that a first end 110a is attached to the top of the meter 120, and a second end 110b is inserted into the base 111 and positioned on the positive y-axis direction side. For example, the subject inserts the right wrist through the space formed by the wearing portion 110, the base 111, and the meter 120 and pulls the second end 110b of the wearing portion 110 in the positive y-axis direction with the left hand, while adjusting the pulse contact portion 132 to contact the skin above the radial artery in the right wrist. The subject pulls the second end 110b until the electronic device 100 is secured on the right wrist and, in this state, secures the wearing portion 110 using the fixing mechanism of the fixing unit 112. In this way, the subject can wear the electronic device 100 with one hand (the left hand in the present embodiment). By securing the electronic device 100 on the wrist using the wearing portion 110, the electronic device 100 can be stabilized in a state being worn. Thus, the positional relationship between the wrist and the electronic device 100 is less likely to change during a measurement, which enables stable measurement of the pulse wave and improves the measurement accuracy.

Next, the movement of a movable portion of the electronic device 100 when the electronic device 100 is worn will be described.

In order to wear the electronic device 100, the subject inserts the wrist into the space formed by the wearing portion 110, the base 111, and the meter 120 along the x-axis direction, as described above. At this time, because the meter 120 is configured to be able to rotate in the directions indicated by the arrow A in FIG. 1 with respect to the base 111, the subject can insert the wrist rotating the meter 120 in the direction indicated by the arrow A. Because the meter 120 is configured to be able to rotate as described above, the subject can insert the wrist appropriately changing the orientation of the meter 120 in accordance with the positional relationship between the subject and the electronic device 100. In this way, the electronic device 100 facilitates wearing of the electronic device 100 for the subject.

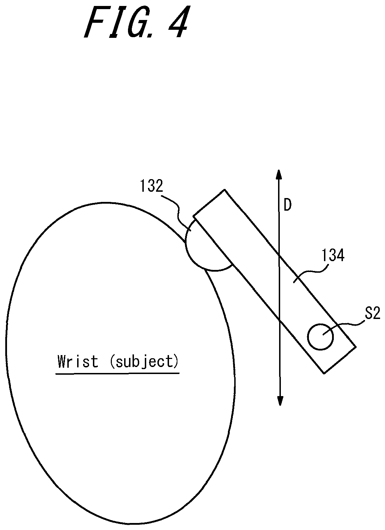

After inserting the wrist into the space formed by the wearing portion 110, the base 111, and the meter 120, the subject brings the pulse contact portion 132 into contact with the skin above the radial artery in the wrist. Here, because the main body 121 can be displaced in the directions indicated by the arrow D in FIG. 1, the first arm 134 of the sensor 130 connected to the main body 121 can also be displaced in the directions indicated by the arrow D, which coincides with the z-axis direction, as illustrated in FIG. 4. Thus, the subject can displace the first arm 134 in the directions indicated by the arrow D in accordance with the width and thickness of the wrist, such that the pulse contact portion 132 contacts the skin above the radial artery. The subject can secure the main body 121 at the displaced position. In this way, the electronic device 100 facilitates adjustment of the position of the sensor 130 to a position appropriate for the measurement. Thus, the electronic device 100 improves the measurement accuracy. Although in the example illustrated in FIG. 1 it is described that the main body 121 can be displaced along the z-axis direction, the main body 121 does not necessarily need to be configured to be displaced along the z-axis direction. The main body 121 simply needs to be configured to allow its positional adjustment in accordance with, for example, a size and a thickness of the wrist. For example, the main body 121 may be configured to be displaced along a direction intersecting the xy plane serving as the surface of the base 111.

Here, when the pulse contact portion 132 contacts the skin above the radial artery in a direction perpendicular to the skin surface, the pulsation transmitted to the first arm 134 is increased. That is, when a displacement direction of the pulse contact portion 132 (the directions indicated by the arrow E in FIG. 3) is perpendicular to the skin surface, the pulsation transmitted to the first arm 134 is increased, and an accuracy in acquiring the pulsation can be improved. In the electronic device 100 according to the present embodiment, the main body 121 and the sensor 130 connected to the main body 121 are configured to be able to rotate about the shaft 124 with respect to the exterior portion 122, as indicated by the arrow C in FIG. 1. Thus, the subject can adjust the orientation of the sensor 130 such that the pulse contact portion 132 is to be displaced in the direction perpendicular to the skin surface. That is, the electronic device 100 can adjust the orientation of the sensor 130 such that the displacement direction of the pulse contact portion 132 becomes perpendicular to the skin surface. In this way, the electronic device 100 can enable adjustment of the orientation of the sensor 130 in accordance with the shape of the wrist of the subject. This configuration facilitates a transfer of a change in the pulsation of the subject. Thus, the electronic device 100 improves the measurement accuracy.

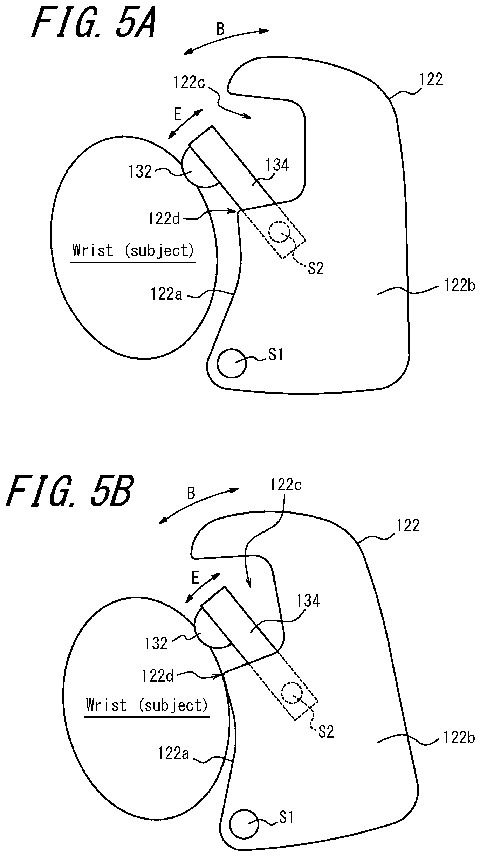

After bringing the pulse contact portion 132 into contact with the skin above the radial artery in the wrist as illustrated in FIG. 5A, the subject pulls the second end 110b of the wearing portion 110 to secure the wrist on the electronic device 100. Here, because the exterior portion 122 can rotate in the directions indicated by the arrow B in FIG. 1, when the subject pulls the wearing portion 110, the exterior portion 122 rotates about the shaft S1, and the top end side of the exterior portion 122 is displaced in the negative y-axis direction. That is, the top end side of the exterior portion 122 is displaced in the negative y-axis direction, as illustrated in FIG. 5B. Because the first arm 134 is connected to the second arm 135 via the elastic member 140, when the top end side of the exterior portion 122 is displaced in the negative y-axis direction, the pulse contact portion 132 is pushed toward the radial artery. This enables the pulse contact portion 132 to more reliably acquire a change in the pulsation. Thus, the electronic device 100 improves the measurement accuracy.

The rotating directions of the exterior portion 122 (the direction indicated by the arrow B) and the rotating directions of the first arm 134 (the direction indicated by the arrow E) may be approximately parallel to each other. As the rotating directions of the exterior portion 122 and the rotating directions of the first arm 134 are closer to parallel to each other, the elastic force of the elastic member 140 more effectively acts on the first arm 134 upon displacement of the top end side of the exterior portion 122 in the negative y-axis direction. Note that a range in which the rotating directions of the exterior portion 122 and the rotating directions of the first arm 134 are approximately parallel to each other includes a range in which the elastic force of the elastic member 140 acts on the first arm 134 upon displacement of the top end side of the exterior portion 122 in the negative y-axis direction.

Here, a surface 122b on the front side of the exterior portion 122 illustrated in FIG. 5A and FIG. 5B has an approximately rectangular shape extending in the up-down direction. The surface 122b has a notch 122c formed on the upper end side in the negative y-axis direction. Because of the notch 122c, when the upper side of the exterior portion 122 is displaced in the negative y-axis direction as illustrated in FIG. 5B, the surface 122b is not likely to contact the skin above the radial artery. Thus, the surface 122b can be easily suppressed from contacting the skin above the radial artery and inhibiting the pulsation of the radial artery.

Further, when the top end side of the exterior portion 122 is displaced in the negative y-axis direction as illustrated in FIG. 5B, the end portion 122d at the bottom of the notch 122c comes into contact with a position different from the position where the radial artery exists in the wrist. Because of the contact of the end portion 122d to the wrist, the exterior portion 122 is not displaced in the negative y-direction exceeding the contact position thereof. Thus, the end portion 122d can suppress displacement of the exterior portion 122 exceeding the predetermined position. If the exterior portion 122 is displaced in the negative y-axis direction exceeding the predetermined position, the first arm 134 is strongly pushed against the skin above the radial artery by the elastic force of the elastic member 140. This may inhibit the pulsation of the radial artery. In the electronic device 100 according to the present embodiment, because the exterior portion 122 includes the end portion 122d, the first arm 134 is suppressed from applying an excessive pressure on the skin above the radial artery, whereby the inhibition of the pulsation of the radial artery can be avoided. Thus, the end portion 122d functions as a stopper for regulating a displacement range of the exterior portion 122.

In the present embodiment, a rotary axis S2 of the first arm 134 may be arranged at a position spaced apart from the negative y-axis direction side of the surface 122b, as illustrated in FIG. 5A and FIG. 5B. When the rotary axis S2 is positioned in the vicinity of the negative y-axis direction side of the surface 122b, the first arm 134 may come into contact with the wrist of the subject and inhibit accurate acquisition of a change in the pulsation of the radial artery. Because the rotary shaft S2 is arranged at a position spaced apart from the negative y-axis direction side of the surface 122b, the probability that the first arm 134 comes into contact with the wrist is reduced, whereby the first arm 134 can more accurately acquire a change in the pulsation.

The subject wears the electronic device 100 on the wrist by pulling the second end 110b of the wearing portion 110 and, in this state, securing the wearing portion 110 using the fixing mechanism of the fixing unit 112. In a state in which the electronic device 100 is worn on the wrist as described above, the first arm 134 is displaced in the directions indicated by the arrow E in accordance with a change in the pulsation, whereby the electronic device 100 measures the pulse wave of the subject.

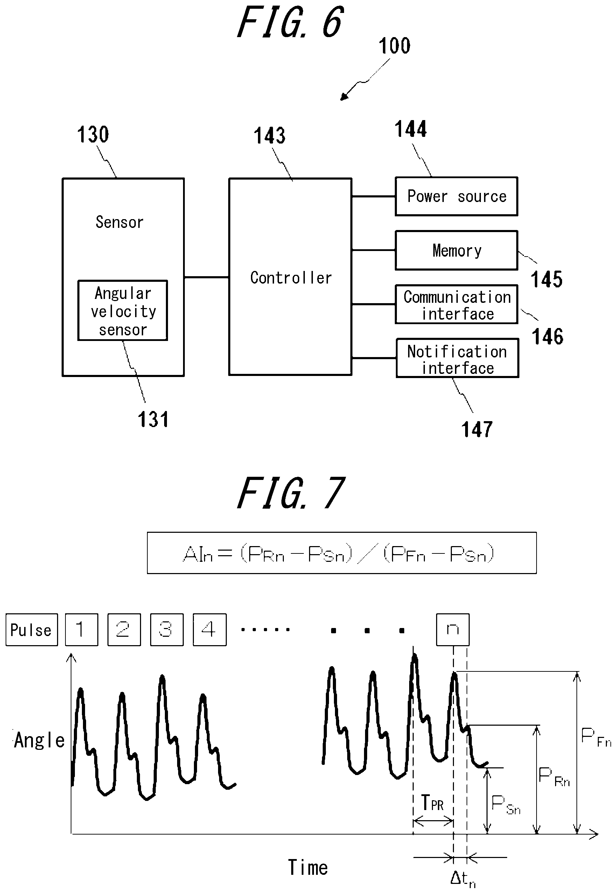

FIG. 6 is a functional block diagram illustrating a schematic configuration of the electronic device 100. The electronic device 100 includes the sensor 130, a controller 143, a power source 144, a memory 145, a communication interface 146, and a notification interface 147. In the present embodiment, the controller 143, the power source 144, the memory 145, the communication interface 146, and the notification interface 147 are enclosed in, for example, the fixing unit 112.

The sensor 130 includes an angular velocity sensor 131 and acquires the pulse wave by detecting the pulsation from the measured part.

The controller 143 is a processor configured to control and manage the electronic device 100 in its entirety including each functional block thereof. Also, the controller 143 is a processor configured to calculate an index based on a propagation phenomenon of a pulse wave from an acquired pulse wave. The controller 143 is a processor such as a CPU (Central Processing Unit) or the like configured to execute a program defining a control procedure and a program for calculating the index based on the propagation phenomenon of a pulse wave. These programs are stored in a storage medium such as, for example, the memory 145. The controller 143 is configured to estimate conditions of the subject, such as a glucose metabolism condition or a lipid metabolism condition, based on a calculated index. The controller 143 is configured to transmit data to the notification interface 147.

The power source 144 includes, for example, a lithium-ion battery and a control circuit for charging and discharging the lithium-ion battery, and supplies electric power to the entire electronic device 100.

The memory 145 stores programs and data. The memory 145 may include any non-transitory storage medium, such as a semiconductor storage medium, a magnetic storage medium, or the like. The memory 145 may include a plurality of types of storage media. The memory 145 may include a combination of a portable storage medium such as a memory card, an optical disc, and a magneto-optical disk, and a reader of the storage medium. The memory 145 may include a storage device to be used as a temporary storage area, such as RAM (Random Access Memory). The memory 145 stores various information and programs for operating the electronic device 100, and functions as a working memory. The memory 145 may store, for example, a pulse wave measurement result acquired by the sensor 130.

The communication interface 146 transmits various data by performing a wired communication or a wireless communication with an external device. The communication interface 146 communicates with, for example, an external device that stores biological information of the subject for the purpose of managing the health condition of the subject, and transmits the pulse wave measurement result acquired by the electronic device 100 and the health condition estimated by the electronic device 100 to the external device.

The notification interface 147 provides notification of information using a sound, a vibration, an image, or the like. The notification interface 147 may include a speaker, a vibrator, or a display device such as a liquid crystal display (LCD: Liquid Crystal Display), an organic EL (OELD: Organic Electroluminescent Display), or an inorganic EL display (IELD: Inorganic Electroluminescent Display). In the present embodiment, the notification interface 147 provides notification of, for example, the glucose metabolism condition or the lipid metabolism condition of the subject.

FIG. 7 is a graph illustrating an example of a pulse wave acquired in the wrist using the electronic device 100. FIG. 7 illustrates a case in which the angular velocity sensor 131 is used as a pulsation detection means. The graph of FIG. 7 is acquired by performing time integration on the angular velocity acquired by the angular velocity sensor 131, and the horizontal axis and the vertical axis represent time and angle, respectively. Because an acquired pulse wave may include a noise caused by, for example, a movement of the body of the subject, a pulsation component alone may be extracted by performing correction on the acquired pulse wave using a filter for removing a DC (Direct Current) component.

A method for calculating an index based on a pulse wave using the acquired pulse wave will be explained with reference to FIG. 7. A pulse wave propagation is a phenomenon in which a beat generated by the blood pumped out from the heart propagates through the arterial wall and blood. The beat generated by the blood pumped out from the heart advances as a advancing wave and reaches peripheries in hands and legs, and a portion of the advancing wave returns as a reflected wave reflected by branches of the blood vessels due to a difference in diameters of the blood vessels, or the like. The index based on the pulse wave is, for example, a pulse-wave propagation velocity PWV (Pulse Wave Velocity) of the advancing wave, P_{R} representing a magnitude of the reflected pulse wave, Δt representing a time difference between the advancing wave of the pulse wave and the reflected wave, an AI (Augmentation Index) expressed by a ratio of the magnitude of the advancing wave of the pulse wave and the magnitude of the reflected wave, or the like.

The pulse wave illustrated in FIG. 7 is the n-number of pulses of a user, where "n" is an integer of 1 or more. The pulse wave is a synthesized wave in which the advancing wave caused by the blood pumped out from the heart and the reflected wave caused by vascular branching or a diameter change of the blood vessel overlap with each other. In FIG. 7, P_{Fn} represents a magnitude of a peak of the pulse wave caused by the advancing wave of each pulse, P_{Rn} represents a magnitude of a peak of the pulse wave caused by the reflection wave of each pulse, and Psn represents a minimum value of the pulse wave for each pulse. In FIG. 7, further, T_{PR} represents a time interval between the peaks of the pulses.

The index based on the pulse wave is acquired by quantifying the information acquired from the pulse wave. For example, the PWV as an index based on the pulse wave is calculated based on a time difference of the pulse wave measured at two measured parts, such as an upper arm and an ankle, and a distance therebetween. In particular, the PWV is acquired by synchronizing pulse waves at two points of the artery (e.g., the upper arm and the ankle) and dividing the distance between the two points (L) by the time difference (PTT) of the pulse waves at the two points. For example, for the magnitude P_{R} of the reflected wave as one of the indices based on the pulse wave, the P_{Rn} representing a magnitude of a peak of a pulse wave by a reflected wave may be calculated, or P_{Rave} acquired by averaging the magnitude of the reflected waves for n-times may be calculated. For example, for the time difference Δt between the advancing wave and the reflected wave serving as one of the indices based on the pulse wave, a time difference Δₜₙ of a predetermined pulse or Δtₐᵥₑ acquired by averaging the time difference for n-times may be calculated. For example, the AI as one of the indices based on the pulse wave is acquired by dividing a magnitude of the reflected wave by a magnitude of the advancing wave, and expressed by AIn = (P_{Rn} - P_{Sn}) / (P_{Fn} -P_{Sn}). The AIₙ represents the AI of each pulse. For the AI, for example, the average AIₐᵥₑ that is acquired by measuring the pulse wave for a few seconds and calculating the average value of the AIn (n is an integer of 1 to n) of each pulse may be used as the index based on the pulse wave.

Because the pulse-wave propagation velocity PWV, the magnitude P_{R} of the reflected wave, the time difference Δt between the advancing wave and the reflected wave, and the AI vary in accordance with the rigidity of the blood vessel wall, they can be used to estimate an arteriosclerosis condition. For example, when the blood vessel wall is rigid, the pulse wave propagation velocity PWV increases. For example, when the blood vessel wall is rigid, the magnitude P_{R} of the reflected wave increases. For example, when the blood vessel wall is rigid, the time difference Δt between the reflected wave and the advancing wave decreases. For example, when the blood vessel wall is rigid, the AI increases. Further, the electronic device 100 can estimate blood fluidity (viscosity) in addition to the arteriosclerosis condition, using the index based on the pulse wave. In particular, the electronic device 100 can estimate a change in the blood fluidity, based on a change in an index based on the pulse wave acquired from the same measured parts of the same subject during a period in which the arteriosclerosis condition remains substantially same (e.g. within a few days). Here, the blood fluidity refers to the degree of easiness of the blood flow. For example, when the blood fluidity is low, the pulse-wave propagation velocity PWV of the reflected wave decreases. For example, when the blood fluidity is low, the magnitude P_{R} of the reflected wave decreases. For example, when the blood fluidity is low, the time difference Δt between the advancing wave and the reflected wave increases. For example, when the blood fluidity is low, the AI decreases.

In the present embodiment, the electronic device 100 calculates the pulse wave propagation velocity PWV, the magnitude P_{R} of the reflected wave, the time difference Δt between the advancing wave and the reflected waves, or the AI, as the index based on the pulse wave, by way of example. However, the index based on the pulse wave is not limited thereto. For example, the electronic device 100 may use a rear systolic blood pressure as the index based on the pulse wave.

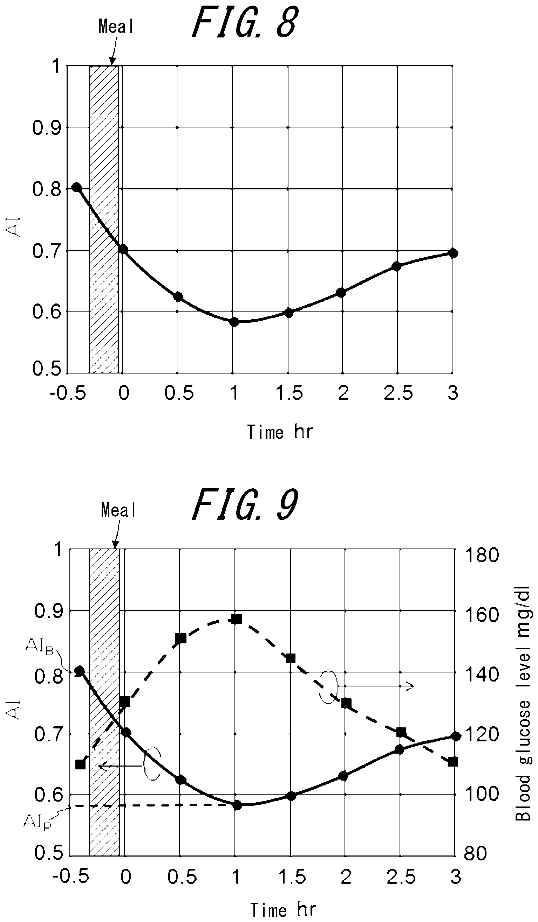

FIG. 8 is a graph illustrating the time variation of the calculated AI. In the present embodiment, the pulse wave was acquired for approximately 5 seconds using the electronic device 100 that includes the angular velocity sensor 131. The controller 143 calculates the AI for each pulse from the acquired pulse wave and also calculates AIₐᵥₑ, the average value thereof. In the present embodiment, the electronic device 100 acquires the pulse wave at a plurality of timings before and after a meal and calculates the average value of AI (hereinafter, referred to as AI) as an example of the index based on the acquired pulse wave. In FIG. 8, the horizontal axis represents elapse of time from 0 representing a first measurement time after the meal, and the vertical axis represents the AI calculated from the pulse wave acquired at the corresponding timing. The pulse wave was acquired above the radial artery of a subject who was in a resting state.

The electronic device 100 acquired the pulse wave before a meal, immediately after the meal, and every 30 minutes after the meal, and calculated a plurality of AI based on the respective pulse waves. The AI calculated from the pulse wave acquired before the meal was approximately 0.8. The AI immediately after the meal was smaller than that before the meal, and the AI at approximately 1 hour after the meal took a minimum extremum value. Then, the AI gradually increased until the end of the measurement at 3 hours after the meal.

The electronic device 100 can estimate a change in the blood fluidity, based on a change in the calculated AI. For example, when red blood cells, white blood cells, or platelets clot forming lumps or increases viscosity, the blood fluidity decreases. For example, when the water content of the plasma in blood decreases, the blood fluidity decreases. Changes in the blood fluidity as described above depend on the health condition of the subject such as, for example, a glycolipids state as will be described later, heat stroke, dehydration, hypothermia, or the like. Before the health condition of the subject becomes severe, the subject can recognize a change in the blood fluidity of the subject using the electronic device 100 of the present embodiment. From the change in the AI before and after the meal illustrated in FIG. 8, it can be estimated that the blood fluidity decreased after the meal, became the lowest at 1 hour after the meal, and gradually increased thereafter. The electronic device 100 may provide notification of a low blood fluidity state by describing it as "Thick", and a high blood fluidity state by describing it as "Thin". For example, the electronic device 100 may determine whether the blood fluidity state is "Thick" or "Thin" referring to an average value of the AI for people at the same age as the subject. When the calculated AI is larger than the average value of the AI, the electronic device 100 may determine the blood fluidity state to be "Thin". When the calculated AI is smaller than the average value of the AI, the electronic device 100 may determine the blood fluidity state to be "Thick". The electronic device 100 may determine whether "Thick" or "Thin", based on the AI before a meal. The electronic device 100 may determine the degree of thickness by comparing the AI after the meal to the AI before the meal. The electronic device 100 can use the AI before a meal, i.e., the AI with the empty stomach as an index of the vascular age (i.e., the rigidity of the blood vessel) of the subject. For example, by calculating a changing amount of the calculated AI using the AI of the subject before the meal, i.e., the AI with the empty stomach as a reference, the electronic device 100 can suppress an estimation error due to the vascular age (the rigidity of the blood vessel) of the subject and thus can more accurately estimate a change in the blood fluidity.

FIG. 9 is a graph illustrating the calculated AI and blood glucose level measurement results. The method for acquiring the pulse wave and the method for calculating the AI are the same as those of the embodiment illustrated in FIG. 8. In FIG. 9, the right vertical axis represents the blood glucose level in the blood, and the left vertical axis represents the calculated AI. In FIG. 9, also, the solid line represents the AI calculated from an acquired pulse wave, and the dotted line represents a measured blood glucose level. The blood glucose level was measured immediately after the pulse wave was acquired. The blood glucose level was measured using a blood glucose meter "Medi-safe Fit" manufactured by Terumo Corporation. The blood glucose level immediately after the meal is higher than that before the meal by approximately 20 mg/dl. The blood glucose level took a maximum extremum value at approximately 1 hour after the meal. Then, the blood glucose level gradually decreased until the measurement was completed and, at approximately 3 hours after the meal, became substantially same as the blood glucose level before the meal.

The blood glucose levels before and after a meal are negatively correlated to the AI calculated from the pulse wave, as illustrated in FIG. 9. When the blood glucose level increases, red blood cells and platelets are clotted by sugar in the blood, or blood increases the viscosity, possibly reducing the blood fluidity as a result. When the blood fluidity decreases, the pulse wave propagation velocity PWV may decrease. When the pulse wave propagation velocity PWV decreases, the time difference between the advancing wave and the reflected wave may increase. When the time difference Δt between the advancing wave and the reflected wave increases, the magnitude P_{R} of the reflected wave may decrease with respect to the magnitude P_{F} of the advancing wave. When the magnitude P_{R} of the reflected wave decreases with respect to the magnitude P_{F} of the advancing wave, the AI may decrease. Since the AI within a few hours after a meal (3 hours in the present embodiment) is correlated to the blood glucose level, a change in the blood glucose level of the subject may be estimated based on a change in the value of the AI. Also, by preliminarily measuring the blood glucose level of the subject and acquiring the correlation to the AI, the electronic device 100 can estimate a blood glucose level of the subject, based on the calculated AI.

The electronic device 100 can estimate a glucose metabolism condition of the subject, based on the occurring time of the minimum extremum value AI_{P}, which is the minimum extremum value of the AI first detected after a meal. The electronic device 100 estimates, for example, the blood glucose level as the glucose metabolism condition. In an example estimation of the glucose metabolism condition, when the minimum extremum value AI_{P} of the AI, which is first detected after a meal, is detected after a predetermined time period (e.g., approximately 1.5 hours or more after a meal), the electronic device 100 can estimate that the subject has a glucose metabolism disorder (i.e., the subject is a diabetic patient).

The electronic device 100 can estimate the glucose metabolism condition of the subject, based on a difference (AI_{B}-AI_{P}) between AI_{B} representing the AI before a meal and the minimum extremum value AI_{P} of the AI first detected after the meal. In an example estimation of the glucose metabolism condition, when the value of (AI_{B}-AI_{P}) is a predetermined value or higher (e.g., 0.5 or more), the electronic device 100 can estimate that the subject has a glucose metabolism abnormality (i.e., the subject is a postprandial hyperglycemia patient).

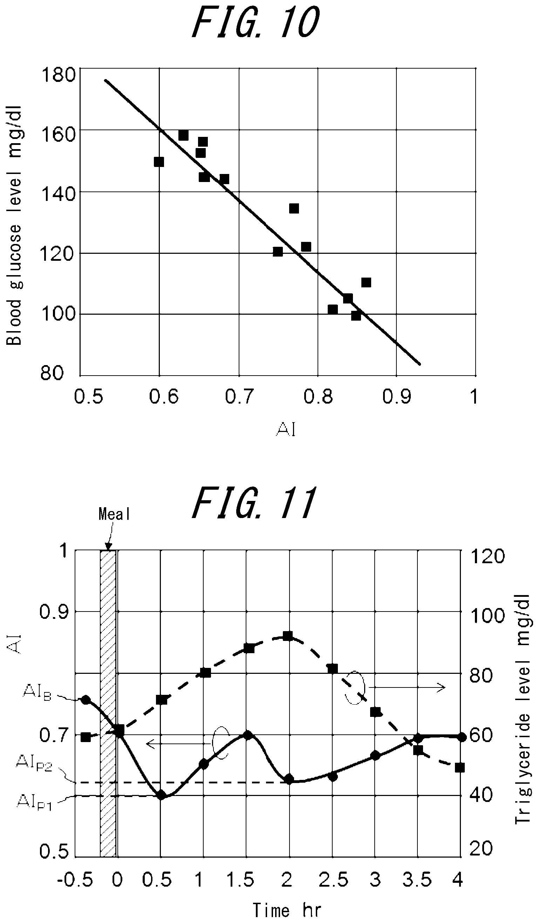

FIG. 10 is a graph illustrating a relationship between the calculated AI and the blood glucose level. The calculated AI and the blood glucose level are acquired within 1 hour after a meal, which is a time period in which the blood glucose level greatly changes. The data in FIG. 10 includes data of the same subject after a plurality of meals. As illustrated in FIG. 10, the calculated AI and the blood glucose level indicated a negative correlation therebetween. A correlation coefficient between the calculated AI and the blood glucose level is 0.9 or more, indicating a very high correlation. For example, by acquiring the correlation between the calculated AI and the blood glucose level as illustrated in FIG. 10 for each subject, the electronic device 100 can estimate the blood glucose level of the subject using the calculated AI.

FIG. 11 is a graph illustrating the calculated AI and triglyceride level measurement results. The method for acquiring the pulse wave and the method for calculating the AI are the same as those of the embodiment illustrated in FIG. 8. In FIG. 11, the right vertical axis indicates the triglyceride level in blood, and the left vertical axis indicates the AI. In FIG. 11, also, the solid line indicates the AI calculated from an acquired pulse wave, and the dotted line indicates a measured triglyceride level. The triglyceride level was measured immediately after the pulse wave was acquired. The triglyceride level was measured using "Pocket Lipid" manufactured by Techno Medica Co., Ltd. The maximum extremum value of the triglyceride level after the meal was higher than that before the meal by approximately 30 mg/dl. The triglyceride level took the maximum extremum value at approximately 2 hours after the meal. Then, the triglyceride level gradually decreased until the measurement was completed and became substantially same as the triglyceride value before the meal at approximately 3.5 hours after the meal.

In contrast, as for the minimum extremum values of the calculated AI, a first minimum extremum value AI_{P1} was detected at approximately 30 minutes after the meal, and a second minimum extremum value AI_{P2} was detected at approximately 2 hours after the meal. It can be estimated that the first minimum extremum value AI _{P1} detected at approximately 30 minutes after the meal is under the influence of the blood glucose level after the meal, as described above. The second minimum extremum value AI_{P2} detected at approximately 2 hours after the meal is approximately concurrent with the maximum extremum value of triglycerides detected at approximately 2 hours after the meal. Thus, it can be estimated that the second minimum extremum value AI_{P2} detected after a predetermined time period from the meal is under the influence of triglycerides. It was found that the triglyceride levels before and after the meal are negatively correlated to the AI calculated from the pulse wave, in a manner similar to the blood glucose level. Especially because the minimum extremum value AI_{P2} of the AI detected after the predetermined time period (approximately 1.5 hours or after in the present embodiment) from the meal is correlated to the triglyceride level, a change in the triglyceride level of the subject can be estimated based on a change in the AI. Also, by preliminarily measuring the triglyceride level of the subject and acquiring its correlation to the AI, the electronic device 100 can estimate the triglyceride level of the subject, based on the calculated AI.

The electronic device 100 can estimate the lipid metabolism condition of the subject, based on the occurrence time of the second minimum extremum value AI_{P2} detected after the predetermined time period from the meal. The electronic device 100 estimates, for example, a lipid level as the lipid metabolism condition. In an example estimation of the lipid metabolism, when the second minimum extremum AI_{P2} is detected after the predetermined time period or later (e.g., more than 4 hours) from the meal, the electronic device 100 can estimate that the subject has abnormal lipid metabolism (i.e., the subject is a hyperlipidemia patient).

The electronic device 100 can estimate the lipid metabolism condition, based on a difference (AI_{B} - AI_{P2}) between AI_{B}, which is the AI before a meal, and the second minimum extremum value AI_{P2} detected after the predetermined time period or later from the meal. In an example estimation of abnormal lipid metabolism, when the difference (AI_{B} - AI_{P2}) is 0.5 or more, the electronic device 100 can estimate that the subject has abnormal lipid metabolism (i.e., the subject is a postprandial hyperlipidemia patient).

Also, from the measurement results illustrated in FIG. 9 to FIG. 11, the electronic device 100 of the present embodiment can estimate the glucose metabolism condition of the subject, based on the first minimum extremum value AI_{P1} first detected after the meal and its occurrence time. Further, the electronic device 100 of the present embodiment can estimate the lipid metabolism condition, based on the second minimum extremum value AI_{P2} detected after a predetermined time period from the detection of the first minimum extremum value AI_{P1}, and the occurrence time of the second minimum extremum value AI_{P2}.

Although triglycerides is used in an estimation example of the lipid metabolism in the present embodiment, the estimation target of the lipid metabolism is not limited to triglycerides. A lipid value estimated by the electronic device 100 includes, for example, total cholesterol, "good" cholesterol (HDL: High-density lipoprotein), or "bad" cholesterol (LDL: Low-density lipoprotein). These lipid levels show a trend similar to that of triglycerides described above.

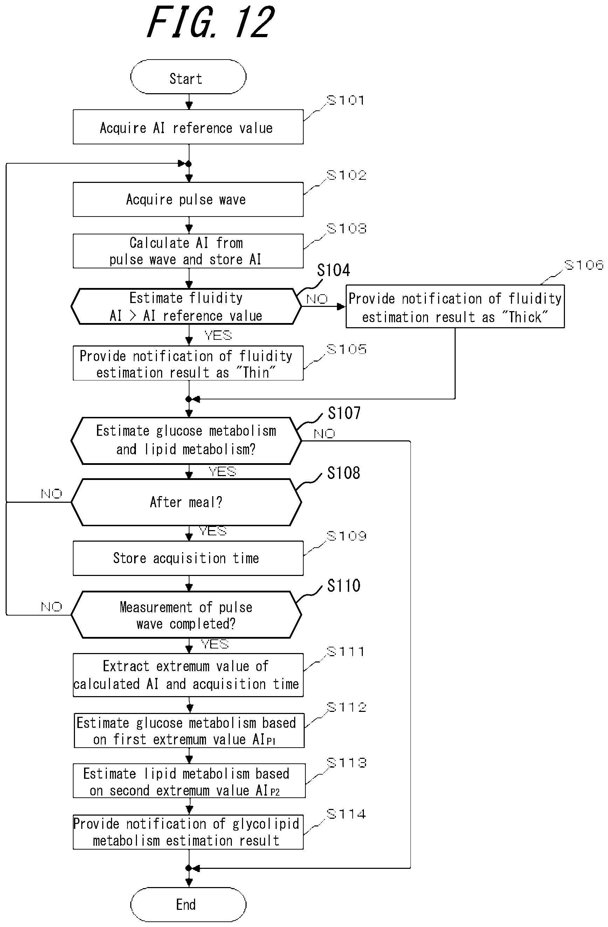

FIG. 12 is a flowchart illustrating a procedure for estimating the blood fluidity, the glucose metabolism condition, and the lipid metabolism condition, based on the AI. The procedure in which the electronic device 100 of the present embodiment estimates the blood fluidity, the glucose metabolism condition, and the lipid metabolism condition will be described with reference to FIG. 12.

As illustrated in FIG. 12, the electronic device 100 acquires an AI reference value of the subject as an initial setting (step S101). The AI reference value may be an average AI estimated from the age of the subject, or the AI of the subject with the empty stomach acquired in advance. Further, the electronic device 100 may use the AI determined as preprandial values in steps S102 to S108, or the AI calculated immediately before a measurement of the pulse wave, as the AI reference value. In this case, the electronic device 100 executes step S101 after steps S102 to S108.

Subsequently, the electronic device 100 acquires the pulse wave (step S102). For example, the electronic device 100 determines whether the pulse wave acquired in a predetermined measuring time (e.g., 5 seconds) has predetermined amplitude or more. In a case in which the acquired pulse wave has the predetermined amplitude or more, the electronic device 100 proceeds to step S103. In a case in which the acquired pulse wave does not have the predetermined amplitude or more, the electronic device 100 repeats step S102 (note that this procedure is not illustrated in the figure). For example, when the electronic device 100 detects the pulse wave having the predetermined amplitude or more in step S102, the electronic device 100 automatically acquires the pulse wave.

The electronic device 100 calculates the AI as an index based on the pulse wave using the pulse wave acquired in step S102, and stores the calculated AI in the memory 145 (step S103). The electronic device 100 may acquire the AI by calculating AIₐᵥₑ, the average value of the AI, from AIn (n is an integer of 1 to n) for each predetermined pulse rate (e.g., 3 beats). Alternatively, the electronic device 100 may calculate the AI of a particular pulse.

The AI may be calculated by performing correction using, for example, a pulse rate (PR), a pulse pressure (PF - PS), body temperature, temperature of the measured part, or the like. It is known that there is a negative correlation between the pulse wave and the AI and between the pulse pressure and the AI, and that there is a positive correlation between the temperature and the AI. In performing the correction, in step S103, for example, the electronic device 100 calculates the pulse rate and a pulse pressure in addition to the AI. For example, the electronic device 100 may include a temperature sensor as the sensor 130 and acquire temperature of the measured part when the pulse wave is acquired in step S102. The AI is corrected by substituting the acquired pulse rate, pulse pressure, temperature, and the like for a preliminarily created correction equation.

Next, the electronic device 100 estimates the blood fluidity of the subject by comparing the AI calculated in step S103 to the AI reference value acquired in step S101 (step S104). In a case in which the calculated AI is greater than the AI reference value (in the case of YES), the electronic device 100 estimates that the blood fluidity is high and provides notification such as, for example, "Blood is thin" (step S105). In a case in which the calculated AI is not greater than the AI reference value (in the case of NO), the electronic device 100 estimates that the blood fluidity is low and provides notification such as, for example, 'Blood is thick" (step S106).

Next, the electronic device 100 asks the subject regarding whether to estimate the glucose metabolism condition and the lipid metabolism condition (step S107). In a case in which the glucose metabolism condition and the lipid metabolism condition are not to be estimated in step S107 (in the case of NO), the electronic device 100 ends the procedure. In a case in which the glucose metabolism condition and the lipid metabolism condition are to be estimated in step S107 (in the case of YES), the electronic device 100 checks whether the calculated AI is acquired before or after a meal (step S108). In a case in which the calculated AI is not a postprandial value (i.e., the calculated AI is acquired before a meal) (in the case of NO), the electronic device 100 returns to step S102 and acquires the next pulse wave. In a case in which the calculated AI is a postprandial value (in the case of YES), the electronic device 100 stores the acquisition time of the pulse wave corresponding to the calculated AI (step S109). In a case in which the pulse wave is to be acquired subsequently (in the case of NO in step S110), the electronic device 100 returns to step S102 and acquires the next pulse wave. In a case in which the measurement of the pulse wave is to be ended (in the case of YES in step S110), the electronic device 100 proceeds to step Sill and the following steps and estimates the glucose metabolism condition and the lipid metabolism condition of the subject.

Next, the electronic device 100 extracts the minimum extremum value and its occurrence time from a plurality of AI calculated in step S103 (step S111). For example, in a case in which the calculated AI show the values as indicated by the solid line in FIG. 11, the electronic device 100 extracts the first minimum extremum value AI_{P1} at approximately 30 minutes after the meal and the second minimum extremum value AI_{P2} at approximately 2 hours after the meal.

Next, the electronic device 100 estimates the glucose metabolism condition of the subject, based on the first minimum extremum value AI_{P1} and its occurrence time (step S112). Further, the electronic device 100 estimates the lipid metabolism condition of the subject, based on the second minimum extremum value AI_{P2} and its occurrence time (step S113). Example estimations of the glucose metabolism condition and lipid metabolism condition of the subject are similar to those described above with reference to FIG. 9 to FIG. 11, and thus descriptions thereof will be omitted.

Next, the electronic device 100 provides notification of the estimation results of the step S112 and step S113 (step S114) and ends the procedure illustrated in FIG. 12. The notification interface 147 provides notification such as, for example, "Glucose metabolism is normal", "Glucose metabolism abnormality is suspected", Lipid metabolism is normal", "Lipid metabolism abnormality is suspected", or the like. Further, the notification interface 147 may provide notification of an advice such as "Medical consultation is advised", "Dietary modification is advised", or the like. Then, the electronic device 100 ends the procedure illustrated in FIG. 12.

In the present embodiment, the electronic device 100 can estimate the blood fluidity, the glucose metabolism condition, and the lipid metabolism condition of the subject using the index based on the pulse wave. Thus, the electronic device 100 can estimate the blood fluidity, the glucose metabolism condition, and the lipid metabolism condition of the subject in a fast and non-invasive manner.

In the present embodiment, the electronic device 100 can estimate the glucose metabolism condition and the lipid metabolism condition using the extremum values of the index based on the pulse wave and their occurrence times. Thus, the electronic device 100 can estimate the glucose metabolism condition and the lipid metabolism condition in a fast and non-invasive manner.

In the present embodiment, the electronic device 100 can estimate the glucose metabolism condition and the lipid metabolism condition of the subject referring to the index based on the pulse wave acquired before a meal (i.e., when the stomach is empty). Thus, the blood fluidity, the glucose metabolism condition, and the lipid metabolism condition of the subject can be accurately estimated without the necessity for regarding the diameter and the rigidity of the blood vessel that do not change in a short time period.

In the present embodiment, the electronic device 100 can estimate the glucose level and the lipid value in a fast and non-invasive manner, by preliminarily performing calibration between the index based on the pulse wave, the blood glucose level, and the lipid level.

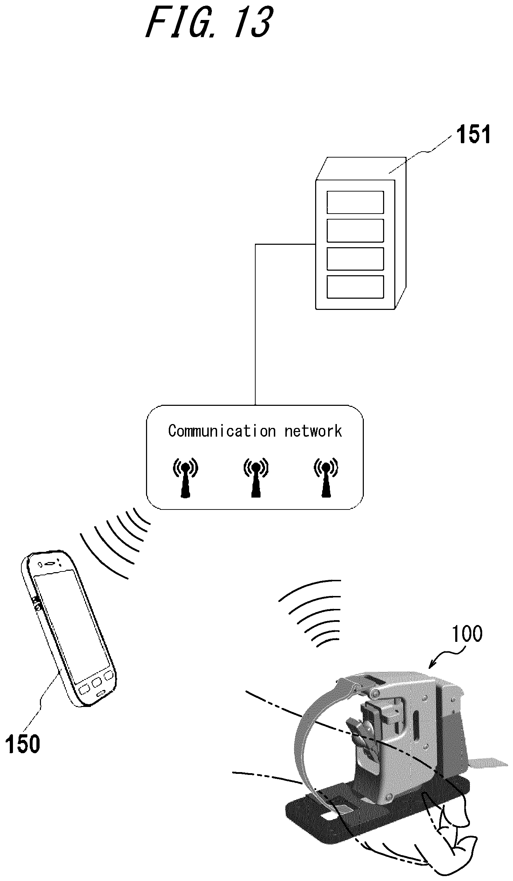

FIG. 13 is a diagram illustrating a schematic configuration of a system according to an embodiment. The system illustrated in FIG. 13 includes the electronic device 100, a server 151, a mobile terminal 150, and a communications network. As illustrated in FIG. 13, an index based on the pulse wave calculated by the electronic device 100 is transmitted to the server 151 via the communication network and stored as personal information of the subject in the server 151. The server 151 estimates the blood fluidity, the glucose metabolism condition, and the lipid metabolism condition of the subject by comparison to past acquired information of the subject and various databases. Further, the server 151 generates an appropriate advice for the subject. The server 151 transmits an estimation result and an advice to the mobile terminal 150 owned by the subject. The mobile terminal 150 provides notification regarding the received estimation result and advice using the display of the mobile terminal 150. In this way, the system functioning as described above can be configured. By using the communication function of the electronic device 100, the server 151 can collect information from a plurality of users and further improve the estimation accuracy. Also, because the mobile terminal 150 is used as a notification means, the electronic device 100 does not need to include the notification interface 147 and can reduce its size. Also, because the server 151 estimates the blood fluidity, the glucose metabolism condition, and the lipid metabolism condition of the subject, the calculation load on the controller 143 of the electronic device 100 can be reduced. Further, because the past acquired information of the subject can be stored in the server 151, the load on the memory 145 of the electronic device 100 can be reduced. Thus, the electronic device 100 can further reduce its size and can be simplified. Also, processing speeds of the operations can be improved.

Although in the system according to the present embodiment the electronic device 100 and the mobile terminal 150 are connected via the communication network using the server 151, the system according to the present disclosure is not limited to such a configuration. In the system, the electronic device 100 and the mobile terminal 150 may be directly connected via the communication network without using the server 151.

Characteristic embodiments have been described in order to completely and clearly disclose the present disclosure. However, the appended claims are not to be construed as being limited to the embodiments described above, and can realize all modifications and alternative configurations that can be created by those skilled in the art within the scope of the basic matters described herein.

For example, although in the embodiment described above the sensor 130 includes the angular velocity sensor 131, the electronic device 100 is not limited to such a configuration. The sensor 130 may include an optical pulse wave sensor equipped with a light emitting unit and a photodetector, or may include a pressure sensor. Also, a wearing position of the electronic device 100 is not limited to the wrist, and the sensor 130 simply needs to be positioned over the artery in the neck, ankle, thigh, ear, or the like.

In the above embodiment, for example, the glucose metabolism condition and the lipid metabolism condition of the subject are estimated based on the first extremum value and the second extremum value, respectively, based on the pulse wave and their occurrence times. However, the operation performed by the electronic device 100 is not limited thereto. There may be a case in which only one of the extremum values appear, or both the extremum values do not appear. In this case, the electronic device 100 may estimate the glucose metabolism condition and the lipid metabolism condition, based on a calculated overall trend (e.g., integral value, Fourier transform, or the like) of time variation of the index based on the pulse wave. The electronic device 100 may estimate the glucose metabolism condition and the lipid metabolism condition, based on a time range in which the index based on the pulse wave falls below a specified value, rather than extracting the extremum values of the index based on the pulse wave.

For example, although in the above embodiment the blood fluidity before and after a meal is estimated, the operation performed by the electronic device 100 is not limited thereto. The electronic device 100 may estimate the blood fluidity before, during, and after exercise, or before, during, and after taking a bath.

In the above embodiment, the natural frequency of the first arm 134 may be set to be close to the frequency of the pulse wave to be acquired. For example, when the frequency of the pulse wave to be acquired is 0.5 to 2 Hz (pulsation: 30 to 120), the first arm 134 may have any natural frequency in a range of 0.5 to 2 Hz. The natural frequency of the first arm 134 can be optimized by varying the length or weight of the first arm 134, or the elastic modulus, the spring constant, or the like of the elastic member 140. By optimizing the natural frequency of the first arm 134, the electronic device 100 can perform measurement more accurately.

Although in the above embodiment the electronic device 100 measures the pulse wave, the pulse wave does not necessarily need to be measured by the electronic device 100. For example, the electronic device 100 may be connected to an information processing apparatus such as a computer or a mobile phone in a wired or wireless manner and may transmit information regarding an angular velocity acquired by the angular velocity sensor 131 to the information processing apparatus. In this case, the information processing apparatus may measure the pulse wave, based on the information regarding the angular velocity. The information processing apparatus may perform the estimation operation of the glucose metabolism condition and the lipid metabolism condition. In a case in which the information processing apparatus connected to the electronic device 100 performs various information processing, the electronic device 100 does not need to include the controller 143, the memory 145, the notification interface 147, or the like. Also, in a case in which the electronic device 100 is connected to the information processing apparatus in a wired manner, the electronic device 100 does not need to include the power source 144 and may receive electric power from the information processing apparatus.

The electronic device 100 does not need to include all of the movable units described in the above embodiments. The electronic device 100 may have only some of the movable units from the movable units described in the above embodiments. For example, the meter 120 does not need to be able to rotate with respect to the base 111. For example, the main body 121 does not need to be displaceable in the up-down direction with respect to the exterior portion 122. For example, the main body 121 does not need to be able to rotate with respect to the exterior portion 122.

In the above embodiment, when the subject pulls the second end 110b of the wearing portion 110, the top end side of the exterior portion 122 is displaced in the negative y-axis direction. However, the exterior portion 122 may be configured such that the top end side thereof is displaced by another mechanism. For example, a mechanism capable of applying a pressure in the negative y-axis direction may be attached to the top end side of the fixing unit 112 so as to push the top end side of the exterior portion 122 in the negative y-axis direction. Such a mechanism can be configured using, for example, a ball screw.

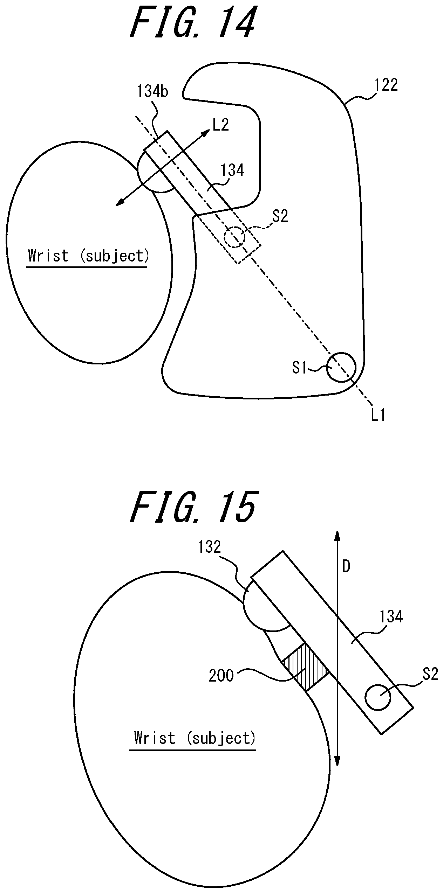

Although in the example illustrated in FIG. 1 the shaft S1 serving as the rotary axis of the exterior portion 122 is arranged on the negative y-axis direction side of the exterior portion 122 in the elevation view, the arrangement of the shaft S1 is not limited thereto. For example, the shaft S1 may be arranged in the vicinity of a straight line L1 connecting the second end 134b, which is an outer peripheral edge of the rotational displacement of the first arm 134, and a shaft S2. For example, the shaft S1 may be arranged on the straight line L1 connecting the second end 134b and the shaft S2, as illustrated in FIG. 14. In an example illustrated in FIG. 14, because the first arm 134 extends to the shaft S2 from the second end 134b, the shaft S1 is arranged on the straight line L1 along which the first arm 134 extends. In a case in which the shaft S1 is arranged on the straight line L1, a displacement direction L2 of the pulse contact portion 132 that rotates about the shaft S2 serving as the rotational axis coincides with the displacement direction of the pulse contact portion 132 that rotates about the shaft S1 serving as the rotational axis. Thus, when the exterior portion 122 is rotated about the shaft S1 serving as the rotational axis, the pulse contact portion 132 is less likely to be shifted from the position on the wrist. As the shaft S1 is located closer to the straight line L1 along which the first arm 134 extends, the contact position of the pulse contact portion 132 on the wrist becomes more unlikely to be shifted by the rotation of the exterior portion 122. Thus, the closer to the straight line L1 the axis S1 is located, the smaller the change in the contact state of the pulse contact portion 132 with respect to the wrist when the subject rotates the exterior portion 122 to secure the electronic device 100 on the wrist. Thus, as the shaft S1 is located closer to the straight line L1, it becomes easier for the subject to wear the electronic device 100 on the wrist while having the pulse contact portion 132 in contact with a desired location.

In the above embodiment, further, the end portion 122d functions as a stopper. In the present disclosure, however, the portion that functions as the stopper is not limited to the end portion 122d. For example, a stopper 200 may be provided to the main body 121, as illustrated in FIG. 15. This stopper 200 may be arranged below the pulse contact portion 132 of the first arm 134. In this case, the stopper moves in conjunction with a movement of the main body 121 in the up-down direction and thus can function as the stopper for subjects including those who have a thin wrist.

### REFERENCE SIGNS LIST

- 100: electronic device
- 110: wearing portion
- 110a, 134a, 135a: first end
- 110b, 134b, 135b: second end
- 111: base
- 112: fixed portion
- 120: meter
- 121: main body
- 122: exterior portion
- 122a: contact surface
- 122b: surface
- 122c: notch
- 122d: end portion
- 123: connecting portion
- 124: shaft
- 125: opening
- 130: sensor
- 131: angular velocity sensor
- 132: pulse contact portion
- 134: first arm
- 135: second arm
- 140: elastic member
- 143: controller
- 144: power source
- 145: memory
- 146: communication interface
- 147: notification interface
- 150: mobile terminal
- 151: server
- 200: stopper

## Claims

1. An electronic device comprising:
a base; and
a meter that can be displaced along a plane intersecting a surface of the base,
wherein the meter includes
an arm that can be displaced in a direction approximately parallel to a displacement direction of the meter in accordance with a pulse wave of a subject, and
a sensor capable of detecting displacement of the arm in accordance with the pulse wave.

2. The electronic device according to claim 1,
wherein displacement of the meter is rotational displacement about a first axis,
the displacement of the arm is rotational displacement about a second axis, and
the first axis is located in a vicinity of a straight line connecting the second axis and an outer peripheral end of the rotational displacement of the arm on a plane orthogonal to the first axis.

3. The electronic device according to claim 1,
wherein the arm can be displaced along a direction intersecting the surface of the base.

4. The electronic device according to claim 1,
wherein the arm can be rotated along the surface of the base with respect to an exterior portion of the meter.

5. The electronic device according to claim 1,
wherein the meter can be rotated along the surface of the base with respect to the base.

6. The electronic device according to claim 1,
wherein the arm further includes a pulse contact portion configured to come into contact with a measured part of the subject.

7. The electronic device according to claim 1, further comprising a wearing portion used to wear the electronic device.

8. The electronic device according to claim 7,
wherein the wearing portion can secure the electronic device on a measured part of the subject.

9. The electronic device according to claim 7,
wherein, when the electronic device is worn using the wearing portion, the meter is displaced along the plane intersecting the surface of the base.

10. The electronic device according to claim 1, further comprising a stopper for regulating a range in which the meter can be displaced.

11. The electronic device according to claim 1, further comprising an elastic member for pushing the arm toward a measured part of the subject when the electronic device is worn.

12. The electronic device according to claim 1,
wherein the sensor detects a change in an angle of the arm in accordance with the pulse wave of the subject.

13. The electronic device according to claim 1,
wherein a natural frequency of the arm is substantially same as a frequency of the pulse wave of the subject.

14. The electronic device according to claim 1,
wherein a natural frequency of the arm is any frequency within a range of 0.5 Hz to 2 Hz.

15. The electronic device according to claim 1, further comprising a controller configured to calculate an index based on a pulse wave acquired by detection of the displacement of the arm by the sensor,
wherein the controller is configured to estimate a glucose metabolism condition or a lipid metabolism condition of the subject, based on the calculated index.

16. The electronic device according to claim 15,
wherein the controller is configured to calculate an index related to a reflected wave from the pulse wave acquired by the sensor, and estimate the glucose metabolism condition or the lipid metabolism condition of the subject, based on the calculated index related to the reflected wave.

17. The electronic device according to claim 1, further comprising a controller configured to calculate an index based on a pulse wave acquired by detection of the displacement of the arm by the sensor,
wherein the controller is configured to estimate blood fluidity of the subject, based on the calculated index.

18. The electronic device according to claim 17,
wherein the controller is configured to calculate an index related to a reflected wave from the pulse wave acquired by the sensor, and estimate the blood fluidity of the subject, based on the calculated index related to the reflected wave.
